# EUROPEAN PATENT SPECIFICATION

(11) **EP 1 846 481 B1**
(45) Date of publication and mention of the grant of the patent: **08.10.2008**
(21) Application number: 06707930.1
(22) Date of filing: 31.01.2006
(51) Int. Cl.: C08J 3/12, C08J 3/075, A61L 15/60

(54) **POLYAMINE-COATED SUPERABSORBENT POLYMERS**
POLYAMIN-BESCHICHTETE SUPERABSORBIERENDE POLYMERE
POLYMERES SUPER ABSORBANTS ENROBES DE POLYAMINE

(30) Priority: 01.02.2005 US 648883 P
(43) Date of publication of application: 24.10.2007
(73) Proprietor: BASF SE, 67056 Ludwigshafen (DE)
(72) Inventor: HERFERT, Norbert, Charlotte, Nc 28226-7200 (US); MIATUDILA, Ma-ikay Kikama, Monroe, Nc 28110 (US); MITCHELL, Michael A., Waxhaw, Nc 28173 (US)
(86) International application number: PCT/EP2006/050563
(87) International publication number: WO 2006/082189

(56) References cited:
- WO-A-97/12575
- US-A- 5 409 771
- US-A- 5 712 316
- US-A- 5 856 410
- PATENT ABSTRACTS OF JAPAN vol. 2002, no. 11, 6 November 2002 (2002-11-06) & JP 2002 212301 A (NIPPON SHOKUBAI CO LTD), 31 July 2002 (2002-07-31)

## Description

### FIELD OF THE INVENTION

The present invention relates to polyamine-coated superabsorbent polymer particles having a reduced tendency to agglomerate and having improved permeability properties. The present invention also relates to methods of manufacturing the polyamine-coated superabsorbent polymer particles from a base superabsorbent polymer particle and a polyammonium carbonate. The polyamine-coated particles exhibit an excellent gel bed permeability essentially without adversely affecting absorption properties. The present invention also relates to the use of the polyamine-coated superabsorbent polymer particles in articles, such as diapers, catamenial devices, and wound dressings.

### BACKGROUND OF THE INVENTION

Water-absorbing resins are widely used in sanitary goods, hygienic goods, wiping cloths, water-retaining agents, dehydrating agents, sludge coagulants, disposable towels and bath mats, disposable door mats, thickening agents, disposable litter mats for pets, condensation-preventing agents, and release control agents for various chemicals. Water-absorbing resins are available in a variety of chemical forms, including substituted and unsubstituted natural and synthetic polymers, such as hydrolysis products of starch acrylonitrile graft polymers, carboxymethylcellulose, crosslinked polyacrylates, sulfonated polystyrenes, hydrolyzed polyacrylamides, polyvinyl alcohols, polyethylene oxides, polyvinylpyrrolidones, and polyacrylonitriles. The most commonly used SAP for absorbing electrolyte-containing aqueous fluids, such as urine, is neutralized polyacrylic acid, e.g., containing about 50% and up to 100%, neutralized carboxyl groups.

Such water-absorbing resins are termed "superabsorbent polymers," or SAPs, and typically are lightly crosslinked hydrophilic polymers. SAPs are generally discussed in Goldman et al. U.S. Patent Nos. 5,669,894 and 5,599,335.

SAPs can differ in their chemical identity, but all SAPs are capable of absorbing and retaining amounts of aqueous fluids equivalent to many times their own weight, even under moderate pressure. For example, SAPs can absorb one hundred times their own weight, or more, of distilled water. The ability to absorb aqueous fluids under a confining pressure is an important requirement for an SAP used in a hygienic article, such as a diaper.

As used herein, the terms "base polymer particles" and "SAP particles" refer to superabsorbent polymer particles in the dry state, i.e., particles containing from no water up to an amount of water less than the weight of the particles. The term "particles" refers to granules, fibers, flakes, spheres, powders, platelets, and other shapes and forms known to persons skilled in the art of superabsorbent polymers. The terms "SAP gel" and "SAP hydrogel" refer to a superabsorbent polymer in the hydrated state, i.e., particles that have absorbed at least their weight in water, and typically several times their weight in water. The term "coated SAP particles" and "coated base polymer particles" refer to particles of the present invention, i.e., SAP particles or base polymer particles, having a polyamine coating.

The terms "surface treated" and "surface crosslinked" refer to an SAP, i.e., base polymer, particle having its molecular chains present in the vicinity of the particle surface crosslinked by a compound applied to the surface of the particle. The term "surface crosslinking" means that the level of functional crosslinks in the vicinity of the surface of the base polymer particle generally is higher than the level of functional crosslinks in the interior of the base polymer particle. As used herein, "surface" describes the outer-facing boundaries of the particle. For porous SAP particles, exposed internal surface also are included in the definition of surface.

The term "polyamine coating" refers to a coating on the surface of an SAP particle, wherein the coating comprises a polymer containing at least two, and typically a plurality, of primary, and/or secondary, and/or tertiary, and/or quaternary nitrogen atoms. The polyamine coating further can comprise an optional inorganic salt having a polyvalent metal cation. The polyvalent metal cation is capable of interacting with nonquaternized nitrogen atoms of the polyamine.

SAP particles can differ in ease and cost of manufacture, chemical identity, physical properties, rate of water absorption, and degree of water absorption and retention, thus making the ideal water-absorbent resin a difficult composition to design. For example, the hydrolysis products of starch-acrylonitrile graft polymers have a comparatively high ability to absorb water, but require a cumbersome process for production and have the disadvantages of low heat resistance and decay or decomposition due to the presence of starch. Conversely, other water-absorbent polymers are easily and cheaply manufactured and are not subject to decomposition, but do not absorb liquids as well as the starch-acrylonitrile graft polymers.

Therefore, extensive research and development has been directed to providing a method of increasing the fluid absorption properties of stable, easy-to-manufacture SAP particles to match the superior fluid absorption properties of difficult-to-manufacture particles. Likewise, it would be advantageous to further increase the fluid absorption properties of already-superior SAP particles.

This is a difficult goal to achieve because improving one desirable property of an SAP particle often adversely affects another desirable property of the SAP particle. For example, absorptivity and gel permeability are conflicting properties. Therefore, a balanced relation between absorptivity and gel permeability is desired in order to provide sufficient liquid absorption, liquid transport, and dryness of the diaper and the skin when using SAP particles in a diaper.

In this regard, not only is the ability of the SAP particles to retain a liquid under subsequent pressure an important property, but absorption of a liquid against a simultaneously acting pressure, i.e., during liquid absorption, also is important. This is the case in practice when a child or adult sits or lies on a sanitary article, or when shear forces are acting on the sanitary article, e.g., leg movements. This absorption property is referred to as absorption under load.

The current trend in the hygiene sector, e.g., in diaper design, is toward ever thinner core constructions having a reduced cellulose fiber content and an increased SAP content. This is an especially important trend in baby diapers and adult incontinence products.

This trend has substantially changed the performance profile required of SAPs. Whereas SAP development initially was focused on very high absorption and swellability, it subsequently was determined that an ability of SAP particles to transmit and distribute a fluid both into the particle and through a bed of SAP particles also is of major importance. Conventional SAPs undergo great surface swelling when wetted with a fluid, such that transport of the fluid into the particle interior is substantially compromised or completely prevented. Accordingly, a substantial amount of cellulose fibers have been included in a diaper core to quickly absorb the fluid for eventual distribution to the SAP particles, and to physically separate SAP particles in order to prevent fluid transport blockage.

An increased amount of SAP particles per unit area in a hygiene article must not cause the swollen polymer particles to form a barrier layer to absorption of a subsequent fluid insult. Therefore, an SAP having good permeability properties ensures optimal utilization of the entire hygiene article. This prevents the phenomenon of gel blocking, which in the extreme case causes the hygiene article to leak. Fluid transmission and distribution, therefore, is of maximum importance with respect to the initial absorption of body fluids.

However, because the absorption properties and permeability properties of SAP particles are conflicting, it is difficult to improve one of these properties without adversely affecting the other property. Investigators have researched various methods of improving the amount of fluid absorbed and retained by SAP particles, especially under load, and the rate at which the fluid is absorbed. One preferred method of improving the absorption and retention properties of SAP particles is to surface treat the SAP particles.

The surface treatment of SAP particles with crosslinking agents having two or more functional groups capable of reacting with pendant carboxylate groups on the polymer comprising the SAP particle is disclosed in numerous patents. Surface treatment improves absorbency and gel rigidity to increase fluid flowability and prevent SAP particle agglomeration, and improves gel strength.

Surface-crosslinked SAP particles, in general, exhibit higher liquid absorption and retention values than SAP particles having a comparable level of internal crosslinks, but lacking surface crosslinks. Internal crosslinks arise from polymerization of the monomers comprising the SAP particles, and are present in the polymer backbone. It has been theorized that surface crosslinking increases the resistance of SAP particles to deformation, thus reducing the degree of contact between surfaces of neighboring SAP particles when the resulting hydrogel is deformed under an external pressure. The degree to which absorption and retention values are enhanced by surface crosslinking is related to the relative amount and distribution of internal and surface crosslinks, and to the particular surface crosslinking agent and method of surface crosslinking.

The present invention is directed to SAP particles, optionally surface treated, that are coated with a polyammonium carbonate, then heated to form polyamine-coated SAP particles. The polyammonium carbonate-coated SAP particles resist a tendency to agglomerate, and the polyamine-coated SAP particles demonstrate an improved gel bed permeability (GBP) without a substantial adverse affect on the fluid absorbency properties of the SAP particles.

### SUMMARY OF THE INVENTION

The present invention is directed to SAP particles having a coating comprising a polyamine. More particularly, the present invention is directed to optionally surface-crosslinked SAP particles having a polyamine coating, and to methods of preparing the polyamine-coated SAP particles using polyammonium carbonate-coated SAP particles as a precursor.

One aspect of the present invention is to provide SAP particles having an excellent gel bed permeability, a high absorbance under load, a good gel strength, and a high centrifuge retention capacity, that also demonstrate an improved ability to absorb and retain electrolyte-containing fluids, such as saline, blood, urine, and menses.

Another aspect of the present invention is to provide polyamine-coated, and optionally surface-crosslinked, SAP particles having the above-listed properties, and a reduced tendency to agglomerate. Polyamine coating (including coating with a polyammonium carbonate) and optional surface crosslinking of the SAP particles can be performed simultaneously or sequentially.

Still another aspect of the present invention is to prepare coated SAP particles of the present invention by (a) applying a polyammmonium carbonate, (b) applying an optional inorganic salt having a polyvalent metal cation, and (c) applying an optional surface-crosslinking agent to the surfaces of SAP particles, followed by heating the resulting SAP particles at about 70°C to 175°C for about 5 to about 90 minutes.

Yet another aspect of the present invention is to provide polyamine-coated, optionally surface-crosslinked, SAP particles having a reduced tendency to agglomerate, i.e., having a reduced tendency to agglomerate compared to identical SAP particles prepared directly from a polyamine rather than a polyammonium carbonate precursor.

Another aspect of the present invention is to provide polyamine-coated, optionally surface-crosslinked, SAP particles having a reduced tendency to agglomerate and an improved permeability, while retaining a high centrifuge retention capacity (CRC) and absorbance under load (AUL).

Yet another aspect of the present invention is to provide polyamine-coated SAP particles having an essentially uniform coating of the polyamine. The uniform coating is achieved by applying a polyammonium carbonate to SAP particles, which also permits applying a reduced amount of a polyammonium carbonate solution to the SAP particles compared to a free polyamine solution. This reduced amount of coating solution facilitates manufacture of the polyamine-coated SAP particles and provides cost savings.

Still another aspect of the present invention is to provide absorbent hygiene articles, such as diapers, having a core comprising polyamine-coated SAP particles of the present invention.

Another aspect of the present invention is to provide absorbent hygiene articles having a core containing a relatively high concentration of polyamine-coated SAP particles, which have a reduced tendency to agglomerate and provide improved permeability essentially without a decrease in absorbent properties.

These and other aspects and advantages of the present invention will become apparent from the following detailed description of the preferred embodiments.

### DETAILED DESCRIPTION OF THE PREFERRED EMBODIMENTS

The present invention is directed to SAP particles coated with a polyamine. The polyamine coating is achieved by applying a polyammonium carbonate to surfaces of the SAP particles, followed by heating of the coated particles to drive off carbon dioxide, generate a polyamine, and form a polyamine coating on the SAP particles.

SAPs for use in personal care products to absorb body fluids are well known. SAP particles typically are polymers of unsaturated carboxylic acids or derivatives thereof. These polymers are rendered water insoluble, but water swellable, by crosslinking the polymer with a di- or polyfunctional internal crosslinking agent. These internally crosslinked polymers are at least partially neutralized and contain pendant anionic carboxyl groups on the polymer backbone that enable the polymer to absorb aqueous fluids, such as body fluids.

SAPs are manufactured by known polymerization techniques, preferably by polymerization in aqueous solution by gel polymerization. The products of this polymerization process are aqueous polymer gels, i.e., SAP hydrogels, that are reduced in size to small particles by mechanical forces, then dried using drying procedures and apparatus known in the art. The drying process is followed by pulverization of the resulting SAP particles to the desired particle size.

To improve the fluid absorption profile, SAP particles are optimized with respect to one or more of absorption capacity, absorption rate, acquisition time, gel strength, and/or permeability. Optimization allows a reduction in the amount of cellulosic fiber in a hygienic article, which results in a thinner article. However, it is difficult to impossible to maximize all of these absorption profile properties simultaneously.

One method of optimizing the fluid absorption profile of SAP particles is to provide SAP particles of a predetermined particle size distribution. In particular, particles too small in size swell after absorbing a fluid and can block the absorption of further fluid. Particles too large in size have a reduced surface area which decreases the rate of absorption.

Therefore, the particle size distribution of the SAP particles is such that fluid permeability, absorption, and retention by the SAP particles is maximized. Any subsequent process that agglomerates the SAP particles to provide oversized particles should be avoided. In particular, agglomeration of SAP particles increases apparent particle size, which reduces the surface area of the SAP particles, and in turn adversely affects absorption of an aqueous fluid by the SAP particles.

The present invention is directed to overcoming problems encountered in improving the absorption profile of SAP particles because improving one property often is detrimental to a second property. The present SAP particles maintain the conflicting properties of a high centrifuge retention capacity (CRC) and an excellent permeability. These problems are overcome in part because of the polyamine coating, and in part because of the reduced tendency of the present coated SAP particles to agglomerate. The present method also provides a method wherein application of a polyamine to the SAP particles is facilitated.

In order to use an increased amount of SAP particles, and a decreased amount of cellulose, in personal care products, it is important to maintain a high liquid permeability. In particular, the permeability of an SAP particle hydrogel layer formed by swelling in the presence of a body fluid is very important to overcome the problem of leakage from the product. A lack of permeability directly impacts the ability of SAP particle hydrogel layers to acquire and distribute body fluids.

Polyamines are known to adhere to cellulose (i.e., fluff), and polyamine-coated SAPs have some improved permeability, as measured in the bulk, for a lower capacity SAP. Coating of SAP particles with uncrosslinked polyamines improves adhesion to cellulose fibers because of the high flexibility of polyamine molecules. However, low molecular weight, uncrosslinked polyamines can be extracted from the SAP particles by wetting with an aqueous fluid. As a result, the viscosity of the aqueous fluid increases, and the acquisition rate of the SAP particles is reduced. If the polyamine is covalently bound to the SAP particles, the degree of SAP particle crosslinking is increased and the absorptive capacity of the particles is reduced. Moreover, covalent bonding of polyamine to the SAP particle surface typically occurs at a temperature greater than 150°C, which adversely affects the color of the SAP particles, and, ultimately, consumer acceptance of the hygiene article.

The addition of a cationic compound, e.g., a polyamine, to improve permeability of SAP particles has been disclosed. WO 03/043670 discloses a polyamine coating on an SAP particle wherein the polyamine molecules are covalently crosslinked to one another. WO 95/22356 and U.S. Patent No. 5,849,405 disclose an absorbent material comprising a mixture of an SAP and an absorbent property modification polymer (e.g., a cationic polymer) that is reactive with at least one component included in urine (e.g., phosphate ion, sulfate ion, or carbonate ion). WO 97/12575 also discloses the addition of a polycationic compound without further crosslinking.

Other patents disclosing incorporation of polyamine-coated superabsorbents in fibrous matrices, e.g., U.S. Patent No. 5,641,561, U.S. Patent No. 5,382,610, EP 0 493 011, and WO 97/39780, relate to an absorbent material having improved structural stability in the dry and wet states. The material comprises water-insoluble hydrogel-forming SAP particles, a polycationic polymer bonded to the absorbent particles at the surface thereof, and glue microfibers that act as an adhesive between SAP particles and the carrier layer. The carrier layer can be a woven or nonwoven material, and the polycationic polymer can be a polyamine, a polyimine, or a mixture thereof. U.S. Patent No. 5,324,561 discloses an SAP which is directly crosslinked to amine-epichlorohydrin adducts (e.g., KYMENE^{®} products).

In accordance with the present invention, optionally surface-crosslinked SAP particles coated with a polyamine, which is applied to the SAP particles via a polyammonium carbonate precursor, are disclosed. The present SAP particles comprise a base polymer. The base polymer can be a homopolymer or a copolymer. The identity of the base polymer is not limited as long as the polymer is an anionic polymer, i.e., contains pendant acid moieties, and is capable of swelling and absorbing at least ten times its weight in water, when in a neutralized form. Preferred base polymers are crosslinked polymers having acid groups that are at least partially in the form of a salt, generally an alkali metal or ammonium salt.

The base polymer typically has at least about 25% of the pendant acid moieties, i.e., carboxylic acid moieties, present in a neutralized form. Preferably, the base polymer has greater than 25% and up to about 100%, and more preferably about 50% up to about 100%, of the pendant acid moieties present in a neutralized form. In accordance with the present invention, the base polymer has a degree of neutralization (DN) of at least 25 to about 100.

The base polymer of the SAP particles is a lightly crosslinked polymer capable of absorbing several times its own weight in water and/or saline. SAP particles can be made by any conventional process for preparing superabsorbent polymers and are well known to those skilled in the art. One process for preparing SAP particles is a solution polymerization method described in U.S. Patent Nos. 4,076,663; 4,286,082; 4,654,039; and 5,145,906. Another process is an inverse suspension polymerization method described in U.S. Patent Nos. 4,340,706; 4,497,930; 4,666,975; 4,507,438; and 4,683,274.

SAP particles useful in the present invention are prepared from one or more monoethylenically unsaturated compound having at least one acid moiety, such as carboxyl, carboxylic acid anhydride, carboxylic acid salt, sulfuric acid, sulfuric acid salt, sulfonic acid, sulfonic acid salt, phosphoric acid, phosphoric acid salt, phosphonic acid, or phosphonic acid salt. SAP particles useful in the present invention preferably are prepared from one or more monoethylenically unsaturated, water-soluble carboxyl or carboxylic acid anhydride containing monomer, and the alkali metal and ammonium salts thereof, wherein these monomers preferably comprise 50 to 99.9 mole percent of the base polymer.

The base polymer of the SAP particles preferably is a lightly crosslinked acrylic resin, such as lightly crosslinked polyacrylic acid. The lightly crosslinked base polymer typically is prepared by polymerizing an acidic monomer containing an acyl moiety, e.g., acrylic acid, or a moiety capable of providing an acid group, i.e., acrylonitrile, in the presence of an internal crosslinking agent, i.e., a polyfunctional organic compound. The base polymer can contain other copolymerizable units, i.e., other monoethylenically unsaturated comonomers, well known in the art, as long as the base polymer is substantially, i.e., at least 10%, and preferably at least 25%, acidic monomer units, e.g., (meth)acrylic acid. To achieve the full advantage of the present invention, the base polymer contains at least 50%, and more preferably, at least 75%, and up to 100%, acidic monomer units. The other copolymerizable units can, for example, help improve the hydrophilicity of the polymer.

Ethylenically unsaturated carboxylic acid and carboxylic acid anhydride monomers useful in the base polymer include acrylic acid, methacrylic acid, ethacrylic acid, α-chloroacrylic acid, α-cyanoacrylic acid, β-methylacrylic acid (crotonic acid), α-phenylacrylic acid, β-acryloxypropionic acid, sorbic acid, α-chlorosorbic acid, angelic acid, cinnamic acid, p-chlorocinnamic acid, β-stearylacrylic acid, itaconic acid, citraconic acid, mesaconic acid, glutaconic acid, aconitic acid, maleic acid, fumaric acid, tricarboxyethylene, and maleic anhydride.

Ethylenically unsaturated sulfonic and phosphonic acid monomers include aliphatic or aromatic vinyl sulfonic acids, such as vinylsulfonic acid, allylsulfonic acid, vinyl toluene sulfonic acid, styrene sulfonic acid, acrylic and methacrylic sulfonic acids, such as sulfoethyl acrylate, sulfoethyl methacrylate, sulfopropyl acrylate, sulfopropyl methacrylate, 2-hydroxy-3-methacryloxypropyl sulfonic acid, 2-acrylamido-2-methylpropane sulfonic acid, vinylphosphonic acid, allylphosphonic acid, and mixtures thereof.

Preferred, but nonlimiting, monomers include acrylic acid, methacrylic acid, maleic acid, fumaric acid, maleic anhydride, and the sodium, potassium, and ammonium salts thereof. An especially preferred monomer is acrylic acid.

The base polymer can contain additional monoethylenically unsaturated monomers that do not bear a pendant acid group, but are copolymerizable with monomers bearing acid groups. Such compounds include, for example, the amides and nitriles of monoethylenically unsaturated carboxylic acids, for example, acrylamide, methacrylamide, acrylonitrile, and methacrylonitrile. Examples of other suitable comonomers include, but are not limited to, vinyl esters of saturated C₁₋₄ carboxylic acids, such as vinyl formate, vinyl acetate, and vinyl propionate; alkyl vinyl ethers having at least two carbon atoms in the alkyl group, for example, ethyl vinyl ether and butyl vinyl ether; esters of monoethylenically unsaturated C₃₋₁₈ alcohols and acrylic acid, methacrylic acid, or maleic acid; monoesters of maleic acid, for example, methyl hydrogen maleate; acrylic and methacrylic esters of alkoxylated monohydric saturated alcohols, for example, alcohols having 10 to 25 carbon atoms reacted with 2 to 200 moles of ethylene oxide and/or propylene oxide per mole of alcohol; and monoacrylic esters and monomethacrylic esters of polyethylene glycol or polypropylene glycol, the molar masses (Mₙ) of the polyalkylene glycols being up to about 2,000, for example. Further suitable comonomers include, but are not limited to, styrene and alkyl-substituted styrenes, such as ethylstyrene and tert-butylstyrene, and 2-hydroxyethyl acrylate.

Polymerization of the acidic monomers, and any copolymerizable monomers, most commonly is performed by free radical processes in the presence of a polyfunctional organic compound. The base polymers are internally crosslinked to a sufficient extent such that the base polymer is water insoluble. Internal crosslinking renders the base polymer substantially water insoluble, and, in part, serves to determine the absorption capacity of the base polymer. For use in absorption applications, a base polymer is lightly crosslinked, i.e., has a crosslinking density of less than about 20%, preferably less than about 10%, and most preferably about 0.01% to about 7%.

A crosslinking agent most preferably is used in an amount of less than about 7 wt%, and typically about 0.1 wt% to about 5 wt%, based on the total weight of monomers. Examples of crosslinking polyvinyl monomers include, but are not limited to, polyacrylic (or polymethacrylic) acid esters represented by the following formula (I), and bisacrylamides represented by the following formula (II): 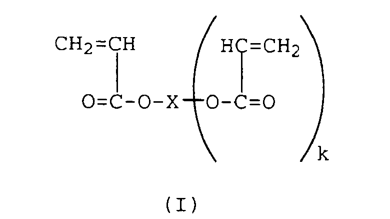 wherein X is ethylene, propylene, trimethylene, cyclohexyl, hexamethylene, 2-hydroxypropylene, -(CH₂CH₂O)ₙCH₂CH₂-, or 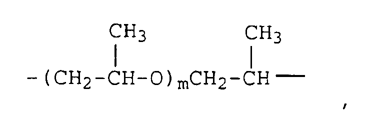 n and m are each an integer 5 to 40, and k is 1 or 2; 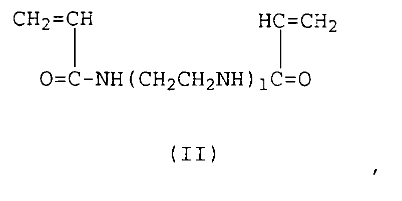 wherein 1 is 2 or 3.

The compounds of formula (I) are prepared by reacting polyols, such as ethylene glycol, propylene glycol, trimethylolpropane, 1,6-hexanediol, glycerin, pentaerythritol, polyethylene glycol, or polypropylene glycol, with acrylic acid or methacrylic acid. The compounds of formula (II) are obtained by reacting polyalkylene polyamines, such as diethylenetriamine and triethylenetetramine, with acrylic acid.

Specific internal crosslinking agents include, but are not limited to, 1,4-butanediol diacrylate, 1,4-butanediol dimethacrylate, 1,3-butylene glycol diacrylate, 1,3-butylene glycol dimethacrylate, diethylene glycol diacrylate, diethylene glycol dimethacrylate, ethoxylated bisphenol A diacrylate, ethoxylated bisphenol A dimethacrylate, ethylene glycol dimethacrylate, 1,6-hexanediol diacrylate, 1,6-hexanediol dimethacrylate, neopentyl glycol dimethacrylate, polyethylene glycol diacrylate, polyethylene glycol dimethacrylate, triethylene glycol diacrylate, triethylene glycol dimethacrylate, tripropylene glycol diacrylate, tetraethylene glycol diacrylate, tetraethylene glycol dimethacrylate, dipentaerythritol pentaacrylate, pentaerythritol tetraacrylate, pentaerythritol triacrylate, trimethylolpropane triacrylate, trimethylolpropane trimethacrylate, tris(2-hydroxyethyl)-isocyanurate triacrylate, ethoxylated trimethylolpropane triacrylate (ETMPTA), e.g., ETMPTA ethyoxylated with 15 moles of ethylene oxide (EO) on average, tris(2-hydroxyethyl)isocyanurate trimethyacrylate, divinyl esters of a polycarboxylic acid, diallyl esters of a polycarboxylic acid, triallyl terephthalate, diallyl maleate, diallyl fumarate, hexamethylenebismaleimide, trivinyl trimellitate, divinyl adipate, diallyl succinate, a divinyl ether of ethylene glycol, cyclopentadiene diacrylate, a tetraallyl ammonium halide, divinyl benzene, divinyl ether, diallyl phthalate, or mixtures thereof. Especially preferred internal crosslinking agents are N,N'-methylenebisacrylamide, N,N'-methylenebismethacrylamide, ethylene glycol dimethacrylate, and trimethylolpropane triacrylate.

The base polymer can be any internally crosslinked polymer having pendant acid moieties that acts as an SAP in its neutralized form. Examples of base polymers include, but are not limited to, polyacrylic acid, hydrolyzed starch-acrylonitrile graft copolymers, starch-acrylic acid graft copolymers, saponified vinyl acetate-acrylic ester copolymers, hydrolyzed acrylonitrile copolymers, hydrolyzed acrylamide copolymers, ethylene-maleic anhydride copolymers, isobutylene-maleic anhydride copolymers, poly(vinylsulfonic acid), poly(vinylphosphonic acid), poly(vinylphosphoric acid), poly(vinylsulfuric acid), sulfonated polystyrene, poly(aspartic acid), poly(lactic acid), and mixtures thereof. The preferred base polymer is a homopolymer or copolymer of acrylic acid or methacrylic acid.

The free radical polymerization is initiated by an initiator or by electron beams acting on a polymerizable aqueous mixture. Polymerization also can be initiated in the absence of such initiators by the action of high energy radiation in the presence of photoinitiators.

Useful polymerization initiators include, but are not limited to, compounds that decompose into free radicals under polymerization conditions, for example, peroxides, hydroperoxides, persulfates, azo compounds, and redox catalysts. Water-soluble initiators are preferred. In some cases, mixtures of different polymerization initiators are used, for example, mixtures of hydrogen peroxide and sodium peroxodisulfate or potassium peroxodisulfate. Mixtures of hydrogen peroxide and sodium peroxodisulfate can be in any proportion.

Examples of suitable organic peroxides include, but are not limited to, acetylacetone peroxide, methyl ethyl ketone peroxide, tert-butyl hydroperoxide, cumeme hydroperoxide, tert-amyl perpivalate, tert-butyl perpivalate, tert-butyl perneohexanoate, tert-butyl perisobutyrate, tert-butyl per-2-ethylhexanoate, tert-butyl perisononanoate, tert-butyl permaleate, tert-butyl perbenzoate, di(2-ethylhexyl) peroxydicarbonate, dicyclohexyl peroxydicarbonate, di(4-tert-butylcyclohexyl) peroxydicarbonate, dimyristyl peroxydicarbonate, diacetyl peroxydicarbonate, an allyl perester, cumyl peroxyneodecanoate, tert-butyl per-3,5,5-trimethyl-hexanoate, acetylcyclohexylsulfonyl peroxide, dilauryl peroxide, dibenzoyl peroxide, and tert-amyl perneodecanoate. Particularly suitable polymerization initiators are water-soluble azo initiators, e.g., 2,2'-azobis(2-amidinopropane) dihydrochloride, 2,2'-azobis(N,N'-dimethylene)isobutyramidine dihydrochloride, 2-(carbamoylazo-isobutyronitrile, 2,2'-azobis[2-(2'-imidazolin-2-yl)propane] dihydrochloride, and 4,4'-azobis(4-cyanovaleric acid). The polymerization initiators are used, for example, in amounts of 0.01% to 5%, and preferably 0.05% to 2.0%, by weight, based on the monomers to be polymerized.

Polymerization initiators also include redox catalysts. In redox catalysts, the oxidizing compound comprises at least one of the above-specified per compounds, and the reducing component comprises, for example, ascorbic acid, glucose, sorbose, ammonium or alkali metal bisulfite, sulfite, thiosulfate, hyposulfite, pyrosulfite, or sulfide, or a metal salt, such as iron (II) ions or sodium hydroxymethylsulfoxylate. The reducing component of the redox catalyst preferably is ascorbic acid or sodium sulfite. Based on the amount of monomers used in the polymerization, about 3 × 10⁻⁶ to about 1 mol% of the reducing component of the redox catalyst system can be used, and about 0.001 to about 5.0 mol% of the oxidizing component of the redox catalyst can be used, for example.

When polymerization is initiated using high energy radiation, the initiator typically comprises a photoinitiator. Photoinitiators include, for example, α-splitters, H-abstracting systems, and azides. Examples of such initiators include, but are not limited to, benzophenone derivatives, such as Michler's ketone; phenanthrene derivatives; fluorene derivatives; anthraquinone derivatives; thioxanthone derivatives; coumarin derivatives; benzoin ethers and derivatives thereof; azo compounds, such as the above-mentioned free-radical formers, substituted hexaarylbisimidazoles, acylphosphine oxides; or mixtures thereof.

Examples of azides include, but are not limited to, 2-(N,N-dimethylamino)ethyl 4-azido-cinnamate, 2-(N,N-dimethylamino)ethyl 4-azido-naphthyl ketone, 2-(N,N-dimethylamino)ethyl 4-azido-benzoate, 5-azido-1-naphthyl 2'-(N,N-dimethylamino)-ethyl sulfone, N-(4-sulfonylazidophenyl)maleimide, N-acetyl-4-sulfonylazidoaniline, 4-sulfonyl-azidoaniline, 4-azidoaniline, 4-azidophenacyl bromide, p-azidobenzoic acid, 2,6-bis(p-azidobenzylidene)cyclohexanone, and 2,6-bis(p-azidobenzylidene)-4-methylcyclohexanone. Photoinitiators customarily are used, if at all, in amounts of about 0.01% to about 5%, by weight of the monomers to be polymerized.

As previously stated, the base polymer is partially neutralized. The degree of neutralization preferably is greater than about 25 mol%, more preferably about 50 to about 100 mol%, most preferably about 70 to about 100 mol%, based on monomers containing acid groups.

Useful neutralizing agents for the base polymer include alkali metal bases, ammonia, and/or amines. Preferably, the neutralizing agent comprises aqueous sodium hydroxide, aqueous potassium hydroxide, or lithium hydroxide. However, neutralization also can be achieved using sodium carbonate, sodium bicarbonate, potassium carbonate, or potassium bicarbonate, or other carbonates or bicarbonates, as a solid or as a solution. Primary, secondary, and/or tertiary amines can be used to neutralize the base polymer.

Neutralization of the base polymer can be performed before, during, or after the polymerization in a suitable apparatus for this purpose. The neutralization is performed, for example, directly in a kneader used for polymerization of the monomers.

In accordance with the present invention, polymerization of an aqueous monomer solution, i.e., gel polymerization, is preferred. In this method, a 10% to 70%, by weight, aqueous solution of the monomers, including the internal crosslinking agent, is neutralized in the presence of a free radical initiator. The solution polymerization is performed at 0°C to 150°C, preferably at 10°C to 100°C, and at atmospheric, superatmospheric, or reduced pressure. The polymerization also can be conducted under a protective gas atmosphere, preferably under nitrogen.

After polymerization, the resulting hydrogel of the base polymer is dried, and the dry base polymer particles are ground and classified to a predetermined size for an optimum fluid absorption profile. In accordance with the present invention, surface crosslinking is optional. However, the base polymer particles typically are surface crosslinked. The base polymer particles first can be surface crosslinked, then coated with a polyammonium carbonate and heated to provide a polyamine coating. Preferably, applying the surface-crosslinking agent onto the base polymer particles is performed simultaneously with applying a polyammonium carbonate.

Polyamines are viscous compounds, and typically are dissolved in a suitable solvent to facilitate handling and allow facile application onto the surfaces of SAP particles. These solutions also may be viscous, which often make it difficult to homogeneously apply a polyamine to SAP surfaces. To overcome this problem, a dilute solution of a polyamine can be used. However, this additional amount of solvent then must be evaporated from the SAP particles, which increases SAP production time and is costly.

In accordance with the present invention, a polyamine is converted into a polyammonium carbonate by passing carbon dioxide through a polyamine solution. The polyammonium carbonate precipitates from the solution to provide a slurry or dispersion of the precipitated polyammonium carbonate. The polyammonium carbonate slurry has a reduced viscosity compared to a polyamine solution, and does not impart a tack to surfaces of the SAP particles.

Therefore, a polyammonium carbonate first is prepared. In one embodiment of preparing polyamine-coated base polymer particles, a coating solution containing the polyammonium carbonate dispersed in a solvent is (are) applied to the surfaces of the base polymer particles. Next, coating solution(s) containing an optional inorganic salt having a polyvalent metal cation and/or an optional surface crosslinking agent dissolved or dispersed in a suitable solvent is applied to the surfaces of the SAP particles. Then, the coated base polymer particles are heated for a sufficient time and at a sufficient temperature to evaporate the solvents of the coating solutions, convert the polyammonium carbonate to a polyamine by driving off carbon dioxide, surface crosslink the base polymer particles (if an optional surface crosslinking agent is used), and form a polyamine coating on the base polymer particles.

It should be understood that the order of applying the polyammonium carbonate, optional inorganic salt, and optional surface crosslinking agent to the surfaces of the base polymer particles is not critical. The components can be added in any order, from two or three solutions. However, the polyammonium carbonate and optional inorganic salt should be applied from different solutions to avoid an interaction prior to application to the base polymer particles.

In another embodiment, the base polymer particles can be surface crosslinked prior to application of the polyammonium carbonate and optional inorganic salt. In still another embodiment, a surface crosslinking agent is applied to the base polymer particles, followed by the polyammonium carbonate and optional inorganic salt, and the particles then are heated to form surface crosslinks and the polyamine coating simultaneously.

In the optional surface crosslinking process, a multifunctional compound capable of reacting with the functional groups of the base polymer is applied to the surface of the base polymer particles, preferably using an aqueous solution. The aqueous solution also can contain water-miscible organic solvents, like an alcohol, such as methanol, ethanol, or i-propanol; a polyol, like ethylene glycol or propylene glycol; or acetone.

A solution of a surface crosslinking agent is applied to the base polymer particles in an amount to wet predominantly only the outer surfaces of the base polymer particles, either before or after application of the polyamine. Surface crosslinking and drying of the base polymer particles then is performed, preferably by heating at least the wetted surfaces of the base polymer particles.

Typically, the base polymer particles are surface treated with a solution of a surface crosslinking agent containing about 0.01% to about 4%, by weight, surface crosslinking agent, and preferably about 0.4% to about 2%, by weight, surface crosslinking agent in a suitable solvent. The solution can be applied as a fine spray onto the surfaces of freely tumbling base polymer particles at a ratio of about 1:0.01 to about 1:0.5 parts by weight base polymer particles to solution of surface crosslinking agent. The surface crosslinking agent, if present at all, is present in an amount of 0.001% to about 5%, by weight of the base polymer particles, and preferably 0.001% to about 0.5% by weight. To achieve the full advantage of the present invention, the surface crosslinking agent is present in an amount of about 0.001% to about 0.1%, by weight of the base polymer particles.

Surface crosslinking and drying of the base polymer particles are achieved by heating the surface-treated base polymer particles at a suitable temperature, e.g., about 70°C to about 150°C, and preferably about 105°C to about 120°C. Suitable surface crosslinking agents are capable of reacting with acid moieties and crosslinking polymers at the surfaces of the base polymer particles.

Nonlimiting examples of suitable surface crosslinking agents include, but are not limited to, an alkylene carbonate, such as ethylene carbonate or propylene carbonate; a polyaziridine, such as 2,2-bishydroxymethyl butanol tris[3-(1-aziridine propionate] or bis-N-aziridinomethane; a haloepoxy, such as epichlorohydrin; a polyisocyanate, such as 2,4-toluene diisocyanate; a di- or polyglycidyl compound, such as diglycidyl phosphonates, ethylene glycol diglycidyl ether, or bischlorohydrin ethers of polyalkylene glycols; alkoxysilyl compounds; polyols such as ethylene glycol, 1,2-propanediol, 1,4-butanediol, glycerol, methyltriglycol, polyethylene glycols having an average molecular weight M_{w} of 200-10,000, di- and polyglycerol, pentaerythritol, sorbitol, the ethoxylates of these polyols and their esters with carboxylic acids or carbonic acid, such as ethylene carbonate or propylene carbonate; carbonic acid derivatives, such as urea, thiourea, guanidine, dicyandiamide, 2-oxazolidinone and its derivatives, bisoxazoline, polyoxazolines, di- and polyisocyanates; di- and poly-N-methylol compounds, such as methylenebis(N-methylolmethacrylamide) or melamine-formaldehyde resins; compounds having two or more blocked isocyanate groups, such as trimethylhexamethylene diisocyanate blocked with 2,2,3,6-tetramethylpiperidin-4-one; and other surface crosslinking agents known to persons skilled in the art.

A solution of the optional surface crosslinking agent is applied to the surfaces of the base polymer particles before or after a solution containing the polyammonium carbonate is applied to the surfaces of the base polymer particles. The polyammonium carbonate also can be applied to the base polymer particles after the surface crosslinking step has been completed.

A dispersion or slurry containing the polyammonium carbonate comprises about 5% to about 50%, by weight, of a polyammonium carbonate in a suitable solvent. The polyammonium carbonate is prepared by passing a sufficient amount of carbon dioxide through a polyamine solution to convert the polyamine to a polyammonium carbonate and form a fine precipitate. Prior to passing carbon dioxide through a polyamine solution, the polyamine is dissolved in a sufficient amount of a solvent to allow the polyammonium carbonate to be readily and homogeneously applied to the surfaces of the base polymer particles. The solvent for the polyamine solution can be, but is not limited to, water, an alcohol, or a glycol, such as methanol, ethanol, ethylene glycol, or propylene glycol, and mixtures thereof.

The amount of polyammonium carbonate applied to the surfaces of the base polymer particles is sufficient to coat the base polymer particle surfaces. Accordingly, the amount of polyammonium carbonate applied to the surfaces of the base polymer particles is about 0.1% to about 2%, and preferably about 0.2% to about 1%, of the weight of the base polymer particle. To achieve the full advantage of the present invention, the polyammonium carbonate is present on the base polymer particle surfaces in an amount of about 0.2% to about 0.5%, by weight of the base polymer particle.

The polyammonium carbonate applied to surfaces of the SAP particles provides tack-free particles. Accordingly, the problem of SAP particle agglomeration is overcome. In addition, the viscosity of polyammonium carbonate dispersion is sufficiently low to permit a facile application of the dispersion onto SAP particle surfaces.

After heating the polyammonium carbonate-coated SAP particles to drive off carbon dioxide and form a polyamine coating on SAP particle surfaces, the resulting polyamine forms an ionic bond with a base polymer and retains adhesive forces to the base polymer after the base polymer absorbs a fluid and swells. Preferably, an excessive amount of covalent bonds are not formed between the polyamine and the base polymer, and the polyamine-base polymer interactions are intermolecular, such as electrostatic, hydrogen bonding, and van der Waals interactions. Therefore, the presence of a polyamine on the base polymer particles does not adversely influence the absorption profile of the base polymer particles.

A polyamine useful in the present invention has at least two, and preferably a plurality, of nitrogen atoms per molecule. The polyamine typically has a weight average molecular weight (M_{w}) of about 5,000 to about 1,000,000, and preferably about 20,000 to about 300,000. To achieve the full advantage of the present invention, the polyamine has an M_{w} of about 100,000 to about 300,000.

In general, useful polyamine polymers have (a) primary amine groups, (b) secondary amine groups, (c) tertiary amine groups, (d) quaternary ammonium groups, or (e) mixtures thereof. Examples of polyamines include, but are not limited to, a polyvinylamine, a polyallylamine, a polyethyleneimine, a polyalkyleneamine, a polyazetidine, a polyvinylguanidine, a poly(DADMAC), i.e., a poly-(diallyl dimethyl ammonium chloride), a cationic polyacrylamide, a polyamine functionalized polyacrylate, and mixtures thereof.

Homopolymers and copolymers of vinylamine also can be used, for example, copolymers of vinylformamide and comonomers, which are converted to vinylamine copolymers. The comonomers can be any monomer capable of copolymerizing with vinylformamide. Nonlimiting examples of such monomers include, but are not limited to, acrylamide, methacrylamide, methacrylonitrile, vinylacetate, vinylpropionate, styrene, ethylene, propylene, N-vinylpyrrolidone, N-vinylcaprolactam, N-vinylimidazole, monomers containing a sulfonate or phosphonate group, vinylglycol, acrylamido(methacrylamido)-alkylene trialkyl ammonium salt, diallyl dialkylammonium salt, C₁₋₄alkyl vinyl ethers such as methyl vinyl ether, ethyl vinyl ether, isopropyl vinyl ether, n-propyl vinyl ether, t-butyl vinyl ether, N-substituted alkyl (meth)acrylamides substituted by a C₁₋₄alkyl group as, for example, N-methylacrylamide, N-isopropylacrylamide, and N,N-dimethylacrylamide, C₁₋₂₀alkyl(meth)acrylic acid esters such as methyl methacrylate, ethyl methacrylate, propyl acrylate, butyl acrylate, hydroxyethyl acrylate, hydroxyethyl methacrylate, hydroxypropyl acrylate, hydroxypropyl methacrylate, hydroxybutyl acrylate, hydroxybutyl methacrylate, 2-methylbutyl acrylate, 3-methylbutyl acrylate, 3-pentyl acrylate, neopentyl acrylate, 2-methylpentyl acrylate, hexyl acrylate, cyclohexyl acrylate, 2-ethylhexyl acrylate, phenyl acrylate, heptyl acrylate, benzyl acrylate, tolyl acrylate, octyl acrylate, 2-octyl acrylate, nonyl acrylate, and octyl methacrylate.

Specific copolymers of polyvinylamine include, but are not limited to, copolymers of N-vinylformamide and vinyl acetate, vinyl propionate, a C₁₋₄alkyl vinyl ether, a (meth)acrylic acid ester, acrylonitrile, acrylamide, and vinylpyrrolidone.

In accordance with the present invention, the number of covalent bonds that form between the polyamine and the base polymer is low, if present at all. However, after heating the polyammonium carbonate-coated SAP particles, the resulting polyamine may impart a tack to surfaces of the base polymer particles, which may lead to agglomeration or aggregation of coated base polymer particles. Therefore, a coating solution containing an optional inorganic salt having a polyvalent metal cation, i.e., a metal cation having a valence of two, three, or four, can be applied to the surfaces of the polyamine-coated base polymer particles.

The polyvalent metal cation is capable of interacting, e.g., forming ionic crosslinks, with the nitrogen atoms of the polyamine. As a result, a tackless polyamine coating is formed on the surface of the base polymer particles. These coated SAP particles have a substantially reduced tendency to agglomerate.

In accordance with the present invention, an optional inorganic salt applied to surfaces of the base polymer particles has a sufficient water solubility such that polyvalent metal cations are available to interact with the nitrogen atoms of the polyamine. Accordingly, a useful inorganic salt has a water solubility of at least 0.1 g of inorganic salt per 100 ml of water, and preferably at least 0.2 g per 100 ml of water.

The polyvalent metal cation of the optional inorganic salt has a valence of +2, +3, or +4, and can be, but is not limited to, Mg²⁺, Ca²⁺, Al³⁺, Sc³⁺, Ti⁴⁺, Mn²⁺, Fe^{2+/3+}, Co²⁺, Ni²⁺, Cu^{+/2+}, Zn²⁺, Y³⁺, Zr⁴⁺, La³⁺, Ce⁴⁺, Hf⁴⁺, Au³⁺, and mixtures thereof. Preferred cations are Mg²⁺, Ca²⁺, Al³⁺, Ti⁴⁺, Zr⁴⁺, La³⁺, and mixtures thereof, and particularly preferred cations are Al³⁺, Ti⁴⁺, Zr⁴⁺, and mixtures thereof. The anion of the inorganic salt is not limited, as long as the inorganic salt has sufficient solubility in water. Examples of anions include, but are not limited to, chloride, bromide, nitrate, and sulfate.

The optional inorganic salt often is present in a coating solution together with an optional surface crosslinking agent. The optional inorganic salt typically is present in a coating solution in an amount of about 0.5% to 20%, by weight, for example. The amount of optional inorganic salt present in a coating solution, and the amount applied to the base polymer particles, is related to the identity of the inorganic salt, its solubility in the solvent of the coating solution, the identity of the polyammonium carbonate to the base polymer particles, and the amount of polyammonium carbonate applied to the base polymer particles. In general, the amount of optional inorganic salt applied to the base polymer particles is sufficient to form a tackless, monolithic polyamine coating and provide coated SAP particles of the present invention.

In accordance with the present invention, the polyammonium carbonate and optional inorganic salt are applied to the base polymer particles in a manner such that each is uniformly distributed on the surfaces of the base polymer particles. Any known method for applying a liquid to a solid can be used, preferably by dispersing a coating solution into fine droplets, for example, by use of a presurized nozzle or a rotating disc. Uniform coating of the base polymer particles can be achieved in a high intensity mechanical mixer or a fluidized mixer which suspends the base polymer particles in a turbulent gas stream. Methods for the dispersion of a liquid onto the surfaces of base polymer particles are known in the art, see, for example, U.S. Patent No. 4,734,478.

Methods of coating the base polymer particles include applying the polyammonium carbonate and inorganic salt simultaneously. The two components preferably are applied via two separate nozzles to avoid interacting before application to the surfaces of the base polymer particles. A preferred method of coating the base polymer is a sequential addition of the components. A more preferred method is a first application of the polyammonium carbonate, followed by an application of the optional inorganic salt. The resulting coated base polymer particles then are heated at about 70°C to about 175°C for sufficient time, e.g., about 5 to about 90 minutes, to generate the polyamine and to cure the polyamine coating.

To demonstrate the unexpected advantages provided by the SAP particles of the present invention, polyamine-coated SAP particles were prepared and tested for centrifuge retention capacity (CRC, g/g), absorbency under load (AUL 0.9 psi, g/g), free swell gel bed permeability (GBP, Darcies), gel bed permeability (GBP 0.3 psi (2.069 kPa), Darcies), and particle size distribution. These tests were performed using the following procedures.

### Centrifuge Retention Capacity (CRC)

This test determines the free swelling capacity of a hydrogel-forming polymer. In this method, 0.2000 ± 0.0050 g of dry SAP particles of size fraction 106 to 850 µm are inserted into a teabag. The teabag is placed in saline solution (i.e., 0.9 wt% aqueous sodium chloride) for 30 minutes (at least 0.83 1 (liter) saline solution/1 g polymer). Then, the teabag is centrifuged for 3 minutes at 250 G. The absorbed quantity of saline solution is determined by measuring the weight of the teabag.

### Free-Swell Gel Bed Permeability (GBP, Darcies)

This procedure is disclosed in U.S. Patent No. 6,387,495.

### Gel Bed Permeability (GBP 0.3 psi, Darcies)

This procedure is identical to that disclosed in U.S. Patent No. 6, 387, 495, except the method is modified by using a 100 gram weight to provide 0.3 psi (2.069 kPa).

### Absorbency Under Load (AUL)

This procedure is disclosed in WO 00/62825, pages 22-23, incorporated herein by reference, using a 317 gram weight for an AUL (0.90 psi) (6.205 kPa).

### Particle Size Distribution

Particle size distribution was determined as set forth in U.S. Patent No. 5,061,259. In summary, a sample of SAP particles is added to the top of a series of stacked sieves. The sieves are mechanically shaken for a predetermined time, then the amount of SAP particles on each sieve is weighed. The percent of SAP particles on each sieve is calculated from the initial sample weight of the SAP sample.

### Example 1

Carbon dioxide is bubbled through a soluion of CATIOFAST^{®} VHF¹⁾ (polyvinylamine, 22% solids). Polyvinylammonium carbonate precipitates from the solution as fine particles. The resulting polyammonium carbonate dispersion can be used as is for application to surfaces of base polymer particles, or can be filtered from the dispersion and applied to the base polymer particles as a solid, or can be diluted with a solvent, e.g., water or an alcohol, to faciltate application of the polyvinylammonium carbonate to surfaces of the base polymer particles. Base polymer²⁾: CRC=30 g/g, particle size distribuion 106-850 µm (amount of particles >850 µm: 0.2 %)

The base polymer (40 g) is coated at room temperature with coating solution 1: 0.66 g of polyvinylammonium carbonate (as active compound in a slurry), 2 g of propylene glycol, and 2.4 g of deionized water, followed by coating with coating solution 2: 0.8 g of deionized water, 0.8 g of propylene glycol, and 0.08 g of ethylene glycol diglycidyl ether (EGDGE). The coated base polymer particles then are cured in a laboratory oven at 150°C for 60 minutes to generate polyvinylamine by expelling carbon dioxide from the polyvinylammonium carbonate, and to cure the polyvinylamine coating on the base polymer particles.

| **CRC (g/g)** | **AUL 0.9 psi (2.069 kPa)** **(g/g)** | **Free Swell** **GBP (Darcies)** | **0.3 psi GBP (6.205 kPa)** **(Darcies)** | **> 850 µm** |
|---|---|---|---|---|
| 23.2 | 18.6 | 235 | 13 | 0.9% |

| | | | | |
|---|---|---|---|---|
| ¹⁾ CATIOFAST^{®} VHF has a molecular weight of about 200,000, and is available from BASF AG, Ludwigshafen, DE; and ²⁾ Base polymer is sodium polyacrylate of DN=74. | | | | |

### Comparative Example 1

Base polymer²⁾ : CRC=30 g/g, particle size distribution 106-850 µm (amount of particles >850 µm: 0.2 %)

The base polymer (40 g) is coated at room temperature with coating solution 1: 3 g of CATIOFAST^{®} VHF¹⁾ (polyvinylamine, 22% solids), and 2 g of propylene glycol, followed by coating with coating solution 2: 0.8 g of deionized water, 0.8 g of propylene glycol, and 0.08 g of EGDGE. The coated base polymer particles are cured in a laboratory oven at 150°C for 60 minutes to cure the polyvinylamine coating on the base polymer particles.

| **CRC (g/g)** | **AUL 0.9 psi (2.069 kPa)** **(g/g)** | **Free Swell** **GBP (Darcies)** | **0.3 psi GBP (6.205 kPa)** **(Darcies)** | **> 850 µm** |
|---|---|---|---|---|
| 24.7 | 16.6 | 206 | 6 | 16.7 |

Example 1 and Comparative Example 1 show that a polyamine coating comprising a polyamine derived from a polyammonium carbonate substantially reduces agglomeration compared to SAP particles coated directly with a polyamine. In addition to reduced agglomeration (>850 µm), permeability is increased (0.3 psi GBP (2.069 kPa) and free swell GBP) and absorbency is maintained (CRC and AUL 0.9 psi (6.205 kPa)).

The coated SAP particles of the present invention are useful as absorbents for water and other aqueous fluids, and can be used as an absorbent component in hygiene articles, such as diapers, tampons, and sanitary napkins. The present polyamine-coated SAP particles also can be used in the following applications, for example: storage, packaging, transportation as a packaging material for water-sensitive articles, for example, flower transportation, and shock protection; food sector for transportation of fish and fresh meat, and the absorption of water and blood in fresh fish and meat packs; water treatment, waste treatment and water removal; cleaning; and agricultural industry in irrigation, retention of meltwater and dew precipitates, and as a composting additive.

Particularly preferred applications for the present polyamine-coated SAP particles include medical uses (wound plaster, water-absorbent material for burn dressings or for other weeping wounds, rapid dressings for injuries, rapid uptake of body fluid exudates for later analytical and diagnostic purposes), cosmetics, carrier material for pharmaceuticals and medicaments, rheumatic plaster, ultrasound gel, cooling gel, thickeners for oil/water or water/oil emulsions, textile (gloves, sportswear, moisture regulation in textiles, shoe inserts, synthetic fabrics), hydrophilicization of hydrophobic surfaces, chemical process industry applications (catalyst for organic reactions, immobilization of large functional molecules (enzymes), heat storage media, filtration aids, hydrophilic component in polymer laminates, dispersants, liquefiers), and building construction (sealing materials, systems or films that self-seal in the presence of moisture, and fine-pore formers in sintered building materials or ceramics).

The present invention also provides for use of the polyamine-coated SAP particles in an absorption core of hygienic articles. The hygienic articles exhibit improved acquisition rates. Hygiene articles include, but are not limited to, incontinence pads and incontinence briefs for adults, diapers for infants, catamenial devices, bandages, and similar articles useful for absorbing body fluids.

Hygiene articles, like diapers, comprise (a) a liquid pervious topsheet; (b) a liquid impervious backsheet; (c) a core positioned between (a) and (b) and comprising 10% to 100% by weight of the present polyamine-coated SAP particles, and 0% to 90% by weight of hydrophilic fiber material; (d) optionally a tissue layer positioned directly above and below said core (c); and (e) optionally an acquisition layer positioned between (a) and (c).

## Claims

1. A superabsorbent polymer particle comprising a base polymer having a surface coating comprising a polyammonium carbonate.

2. The superabsorbent polymer particle of claim 1 wherein the surface coating further comprises an inorganic salt having a polyvalent metal cation.

3. The superabsorbent polymer particle of claim 1 wherein the particle is surface crosslinked.

4. The superabsorbent polymer particle of claim 1 wherein the base polymer contains a plurality of pendant neutralized and unneutralized carboxylic acid groups.

5. The superabsorbent polymer particle of claim 1 wherein the base polymer has a degree of neutralization from 25 to 100.

6. The superabsorbent polymer particle of claim 1 wherein the base polymer comprises acrylic acid, methacrylic acid, or a mixture thereof.

7. The superabsorbent polymer particle of claim 1 wherein the polyammonium carbonate is present on surfaces of the base polymer in an amount of 0.1% to 2%, by weight of the particle.

8. The superabsorbent polymer particle of claim 1 wherein the polyammonium carbonate is based on a polyamine having one or more of primary amino groups, secondary amino groups, tertiary amino groups, and quaternary ammonium groups.

9. The superabsorbent polymer particle of claim 1 wherein the polyammonium carbonate has a weight average molecular weight of 5,000 to 1,000,000.

10. The superabsorbent polymer particle of claim 1 wherein the polyammonium carbonate is prepared from a homopolymer or a copolymer selected from the group consisting of a polyvinylamine, a polyethyleneimine, a polyallylamine, a polyalkyleneamine, a polyazetidine, a polyvinylguanidine, a poly(DADMAC), a cationic polyacrylamide, a polyamine functionalized polyacrylate, and mixtures thereof.

11. The superabsorbent polymer particle of claim 2 wherein the inorganic salt has a water solubility of at least 0.1 g per 100 g of water at 25°C.

12. The superabsorbent polymer particle of claim 2 wherein the polyvalent metal cation of the inorganic salt has a valence of +2, +3, or +4.

13. The superabsorbent polymer particle of claim 2 wherein the polyvalent metal cation of the inorganic salt is selected from the group consisting of Mg²⁺, Ca²⁺, Al³⁺, Sc³⁺, Ti⁴⁺, Mn²⁺, Fe^{2+/3+}, Co²⁺, Ni²⁺, Cu^{+/2+}, Zn²⁺, Y³⁺, Zr⁴⁺, La³⁺, Ce⁴⁺, Hf⁴⁺, Au³⁺, and mixtures thereof.

14. The superabsorbent polymer particle of claim 1 wherein the base polymer comprises a polyacrylic acid.

15. The superabsorbent polymer particle of claim 14 wherein the polyammonium carbonate comprises a homopolymer or a copolymer of polyvinylammonium carbonate.

16. The superabsorbent polymer particle of claim 14 wherein the base polymer is surface crosslinked.

17. The superabsorbent polymer particle of claim 14 wherein surface coating further comprises an inorganic salt having a polyvalent metal cation.

18. The superabsorbent polymer particle of claim 17 wherein the polyvalent metal cation comprises Al³⁺.

19. The superabsorbent polymer particle of claim 1 wherein the particle is heat treated.

20. A method of preparing a superabsorbent polymer particle comprising:
(a) providing a base polymer particle;
(b) providing a polyammonium carbonate;
(c) applying the polyammonium carbonate to surfaces of the base polymer particle;
(d) optionally applying an inorganic salt having a polyvalent metal cation to the surfaces of the base polymer particle;
(e) optionally applying a surface crosslinking agent to the surfaces of the base polymer.

21. The method of claim 20 further comprising:
(f) heating the coated base polymer resulting from steps (c), (d), and (e) at a sufficient temperature and for a sufficient time to provide a cured polyamine coating on the base polymer particle.

22. The method of claim 21 wherein the heating step (f) is performed at 70°C to 175°C for about 5 minutes to 90 minutes.

23. The method of claim 20 wherein step (c) is performed prior to step (e).

24. The method of claim 20 wherein step (c) is performed after step (e).

25. The method of claim 20 wherein step (d) is performed prior to step (e).

26. The method of claim 20 wherein step (d) and step (e) are performed simultaneously.

27. The method of claim 20 wherein step (c) and step (d) are performed simultaneously.

28. The method of claim 20 wherein step (c) and step (e) are performed simultaneously.

29. A method preparing a superabsorbent polymer particle having a polyamine coating comprising:
(a) providing a surface-crosslinked base polymer particle;
(b) providing a polyammonium carbonate;
(c) applying the polyammonium carbonate to surfaces of the base polymer particle;
(d) optionally applying an inorganic salt having a polyvalent metal cation to the surfaces of the base polymer particle;
(e) heating the coated base polymer resulting from steps (c) and (d) at a sufficient temperature and for a sufficient time to provide a cured polyamine coating on the surface-crosslinked base polymer particle.

## Patentansprüche

1. Superabsorberpartikel, umfassend ein Basispolymer mit einer ein Polyammoniumcarbonat umfassenden Oberflächenbeschichtung.

2. Superabsorberpartikel nach Anspruch 1, bei dem die Oberflächenbeschichtung zusätzlich ein anorganisches Salz mit einem mehrwertigen Metallkation umfaßt.

3. Superabsorberpartikel nach Anspruch 1, bei dem das Partikel oberflächenvernetzt ist.

4. Superabsorberpartikel nach Anspruch 1, bei dem das Basispolymer mehrere anhängige neutralisierte und nichtneutralisierte Carbonsäuregruppen enthält.

5. Superabsorberpartikel nach Anspruch 1, bei dem das Basispolymer einen Neutralisationsgrad von 25 bis 100 aufweist.

6. Superabsorberpartikel nach Anspruch 1, bei dem das Basispolymer Acrylsäure, Methacrylsäure oder eine Mischung davon umfaßt.

7. Superabsorberpartikel nach Anspruch 1, bei dem das Polyammoniumcarbonat in einer auf das Gewicht des Partikels bezogenen Menge von 0,1% bis 2% auf Oberflächen des Basispolymers vorliegt.

8. Superabsorberpartikel nach Anspruch 1, bei dem das Polyammoniumcarbonat auf einem primäre Aminogruppen, sekundäre Aminogruppen, tertiäre Aminogruppen und quaternäre Ammoniumgruppen ein- oder mehrfach, gleich oder verschieden aufweisenden Polyamin basiert.

9. Superabsorberpartikel nach Anspruch 1, bei dem das Polyammoniumcarbonat ein gewichtsmittleres Molekulargewicht von 5.000 bis 1.000.000 aufweist.

10. Superabsorberpartikel nach Anspruch 1, bei dem das Polyammoniumcarbonat hergestellt ist aus einem Homopolymer oder einem Copolymer, ausgewählt aus der Gruppe, bestehend aus einem Polyvinylamin, einem Polyethylenimin, einem Polyallylamin, einem Polyalkylenamin, einem Polyazetidin, einem Polyvinylguanidin, einem Poly (DADMAC), einem kationischen Polyacrylamid, einem mit Polyamin funktionalisiertem Polyacrylat und deren Mischung.

11. Superabsorberpartikel nach Anspruch 2, bei dem das anorganische Salz eine Wasserlöslichkeit von mindestens 0,1 g pro 100 g Wasser bei 25°C aufweist.

12. Superabsorberpartikel nach Anspruch 2, bei dem das mehrwertige Metallkation des anorganischen Salzes eine Wertigkeit von +2, +3 oder +4 aufweist.

13. Superabsorberpartikel nach Anspruch 2, bei dem das mehrwertige Metallkation des anorganischen Salzes ausgewählt ist aus der Gruppe, bestehend aus Mg²⁺, Ca²⁺, Al³⁺, Sc³⁺, Ti⁴⁺, Mn²⁺, Fe^{2+/3+}, Co²⁺, Ni²⁺, Cu^{+/2+}, Zn²⁺, Y³⁺, Zr⁴⁺, La³⁺, Ce⁴⁺, Hf⁴⁺, Au³⁺ und deren Mischungen.

14. Superabsorberpartikel nach Anspruch 1, bei dem das Basispolymer eine Polyacrylsäure umfaßt.

15. Superabsorberpartikel nach Anspruch 14, bei dem das Polyammoniumcarbonat ein Homopolymer oder ein Copolymer von Polyvinylammoniumcarbonat umfaßt.

16. Superabsorberpartikel nach Anspruch 14, bei dem das Basispolymer oberflächenvernetzt ist.

17. Superabsorberpartikel nach Anspruch 14, bei dem die Oberflächenbeschichtung zusätzlich ein anorganisches Salz mit einem mehrwertigen Metallkation umfaßt.

18. Superabsorberpartikel nach Anspruch 17, bei dem das mehrwertige Metallkation Al³⁺ umfaßt.

19. Superabsorberpartikel nach Anspruch 1, bei dem das Partikel getempert ist.

20. Verfahren zur Herstellung eines Superabsorberpartikels, bei dem man
(a) ein Basispolymerpartikel bereitstellt,
(b) ein Polyammoniumcarbonat bereitstellt,
(c) das Polyammoniumcarbonat auf Oberflächen des Basispolymerpartikels aufträgt,
(d) gegebenenfalls ein anorganisches Salz mit einem mehrwertigen Metallkation auf Oberflächen des Basispolymerpartikels aufträgt,
(e) gegebenenfalls ein Oberflächenvernetzungsmittel auf die Oberflächen des Basispolymers aufträgt.

21. Verfahren nach Anspruch 20, bei dem man ferner
(f) das aus den Schritten (c), (d) und (e) resultierende beschichtete Basispolymer bei einer ausreichenden Temperatur ausreichend lange erhitzt, um eine gehärtete Polyaminbeschichtung auf dem Basispolymerpartikel bereitzustellen.

22. Verfahren nach Anspruch 21, bei dem das Erhitzen (f) bei 70°C bis 175°C etwa 5 Minuten bis 90 Minuten lang erfolgt.

23. Verfahren nach Anspruch 20, bei dem man Schritt (c) vor Schritt (e) durchführt.

24. Verfahren nach Anspruch 20, bei dem man Schritt (c) nach Schritt (e) durchführt.

25. Verfahren nach Anspruch 20, bei dem man Schritt (d) vor Schritt (e) durchführt.

26. Verfahren nach Anspruch 20, bei dem man Schritt (d) und Schritt (e) gleichzeitig durchführt.

27. Verfahren nach Anspruch 20, bei dem man Schritt (c) und Schritt (d) gleichzeitig durchführt.

28. Verfahren nach Anspruch 20, bei dem man Schritt (c) und Schritt (e) gleichzeitig durchführt.

29. Verfahren zur Herstellung eines Superabsorberpartikels mit einer Polyaminbeschichtung, bei dem man
(a) ein oberflächenvernetztes Basispolymerpartikel bereitstellt,
(b) ein Polyammoniumcarbonat bereitstellt,
(c) das Polyammoniumcarbonat auf Oberflächen des Basispolymerpartikels aufträgt,
(d) gegebenenfalls ein anorganisches Salz mit einem mehrwertigen Metallkation auf die Oberflächen des Basispolymerpartikels aufträgt,
(e) das aus den Schritten (c) und (d) resultierende beschichtete Basispolymer bei einer ausreichenden Temperatur ausreichend lange erhitzt, um eine gehärtete Polyaminbeschichtung auf dem oberflächenvernetzten Basispolymerpartikel bereitzustellen.

## Revendications

1. Particule de polymère super-absorbante comprenant un polymère de base présentant un revêtement de surface comprenant un carbonate de polyammonium.

2. Particule de polymère super-absorbante selon la revendication 1 dans laquelle le revêtement de surface comprend, en outre, un sel inorganique renfermant un cation métallique polyvalent.

3. Particule de polymère super-absorbante selon la revendication 1, la particule étant réticulée en surface.

4. Particule de polymère super-absorbante selon la revendication 1 dans laquelle le polymère de base renferme une pluralité de groupes acide carboxylique pendants, neutralisés et non neutralisés.

5. Particule de polymère super-absorbante selon la revendication 1 dans laquelle le polymère de base présente un degré de neutralisation de 25 à 100.

6. Particule de polymère super-absorbante selon la revendication 1 dans laquelle le polymère de base comprend l'acide acrylique, l'acide méthacrylique, ou un mélange de ceux-ci.

7. Particule de polymère super-absorbante selon la revendication 1 dans laquelle le carbonate de polyammonium est présent sur des surfaces du polymère de base en une quantité de 0,1 % à 2 % en poids de la particule.

8. Particule de polymère super-absorbante selon la revendication 1 dans laquelle le carbonate de polyammonium est à base d'une polyamine renfermant un ou plusieurs parmi des groupes amino primaires, des groupes amino secondaires, des groupes amino tertiaires et des groupes ammonium quaternaires.

9. Particule de polymère super-absorbante selon la revendication 1 dans laquelle le carbonate de polyammonium présente un poids moléculaire moyen en poids de 5 000 à 1 000 000.

10. Particule de polymère super-absorbante selon la revendication 1 dans laquelle le carbonate de polyammonium est préparé à partir d'un homopolymère ou d'un copolymère choisi parmi le groupe constitué d'une polyvinylamine, d'une polyéthylène-imine, d'une polyallylamine, d'une polyalkylène-amine, d'une polyazétidine, d'une polyvinylguanidine, d'un poly(DADMAC), d'un polyacrylamide cationique, d'un polyacrylate à fonction polyamine, et de leurs mélanges.

11. Particule de polymère super-absorbante selon la revendication 2 dans laquelle le sel inorganique présente une solubilité dans l'eau d'au moins 0,1 g pour 100 g d'eau à 25 °C.

12. Particule de polymère super-absorbante selon la revendication 2 dans laquelle le cation métallique polyvalent du sel inorganique présente une valence de +2, +3 ou +4.

13. Particule de polymère super-absorbante selon la revendication 2 dans laquelle le cation métallique polyvalent du sel inorganique est choisi parmi le groupe constitué du Mg²⁺, du Ca²⁺, de l'Al³⁺, du SC³⁺, du Ti⁴⁺, du Mn²⁺, du Fe^{2+/3+}, du Co²⁺, du Ni²⁺ du Cu^{+/2+}, du Zn²⁺, de l'Y³⁺, du Zr⁴⁺, du La³⁺, du Ce⁴⁺, de l'Hf⁴⁺, de l'Au³⁺, et de leurs mélanges.

14. Particule de polymère super-absorbante selon la revendication 1 dans laquelle le polymère de base comprend un acide polyacrylique.

15. Particule de polymère super-absorbante selon la revendication 14 dans laquelle le carbonate de polyammonium comprend un homopolymère ou un copolymère de carbonate de polyvinylammonium.

16. Particule de polymère super-absorbante selon la revendication 14 dans laquelle le polymère de base est réticulé en surface.

17. Particule de polymère super-absorbante selon la revendication 14 dans laquelle le revêtement en surface comprend, en outre, un sel inorganique renfermant un cation métallique polyvalent.

18. Particule de polymère super-absorbante selon la revendication 17 dans laquelle le cation métallique polyvalent comprend l'Al³⁺.

19. Particule de polymère super-absorbante selon la revendication 1, la particule étant soumise à un traitement athermique.

20. Procédé de préparation d'une particule de polymère super-absorbante comprenant les étapes consistant à :
(a) procurer une particule de polymère de base ;
(b) procurer un carbonate de polyammonium ;
(c) appliquer le carbonate de polyammonium sur des surfaces de la particule de polymère de base ;
(d) appliquer, éventuellement, un sel inorganique renfermant un cation métallique polyvalent sur les surfaces de la particule de polymère de base ;
(e) appliquer, éventuellement, un agent de réticulation en surface sur les surfaces du polymère de base.

21. Procédé selon la revendication 20 comprenant, en outre, l'étape consistant à :
(f) chauffer le polymère de base revêtu, résultant des étapes (c), (d) et (e), à une température suffisante et pendant une période suffisante pour procurer un revêtement de polyamine durci sur la particule de polymère de base.

22. Procédé selon la revendication 21 dans lequel l'étape de chauffage (f) est réalisée entre 70°C et 175 °C pendant environ 5 minutes à 90 minutes.

23. Procédé selon la revendication 20 dans lequel l'étape (c) est réalisée avant l'étape (e).

24. Procédé selon la revendication 20 dans lequel l'étape (c) est réalisée après l'étape (e).

25. Procédé selon la revendication 20 dans lequel l'étape (d) est réalisée avant l'étape (e).

26. Procédé selon la revendication 20 dans lequel l'étape (d) et l'étape (e) sont réalisées simultanément.

27. Procédé selon la revendication 20 dans lequel l'étape (c) et l'étape (d) sont réalisées simultanément.

28. Procédé selon la revendication 20 dans lequel l'étape (c) et l'étape (e) sont réalisées simultanément.

29. Procédé de préparation d'une particule de polymère super-absorbante présentant un revêtement de polyamine, comprenant les étapes consistant à :
(a) procurer une particule de polymère de base réticulée en surface ;
(b) procurer un carbonate de polyammonium ;
(c) appliquer le carbonate de polyammonium sur des surfaces de la particule de polymère de base ;
(d) appliquer, éventuellement, un sel inorganique renfermant un cation métallique polyvalent sur les surfaces de la particule de polymère de base ;
(e) chauffer le polymère de base revêtu, résultant des étapes (c) et (d), à une température suffisante et pendant une période suffisante pour procurer un revêtement de polyamine durci sur la particule de polymère de base réticulée en surface.
